# FASCICULE DE BREVET EUROPEEN

(11) **EP 2 558 164 B1**
(45) Date de publication et mention de la délivrance du brevet: **17.12.2014**
(21) Numéro de dépôt: 11718445.7
(22) Date de dépôt: 01.04.2011
(51) Int. Cl.: A61N 5/10, G21K 1/02

(54) **DISPOSITIF DE GUIDAGE DE PHOTONS POUR APPAREIL DE RADIOTHERAPIE**
FOTONENLENKVORRICHTUNG FÜR EIN STRAHLENTHERAPIEGERÄT
PHOTON-GUIDING DEVICE FOR A RADIOTHERAPY APPARATUS

(30) Priorité: 15.04.2010 FR 1052870
(43) Date de publication de la demande: 20.02.2013
(73) Titulaire: Kerjean, Joël, 44800 Saint-Herblain (FR)
(72) Inventeur: Kerjean, Joël, 44800 Saint-Herblain (FR)
(74) Mandataire: Laget, Jean-Loup
(86) Numéro de dépôt international: PCT/FR2011/050733
(87) Numéro de publication internationale: WO 2011/128550

(56) Documents cités:
- WO-A1-2005/015572
- FR-A- 1 561 351
- US-A1- 2003 209 677
- US-B2- 6 749 300

## Description

L'invention concerne un dispositif de guidage de photons pour appareil de radiothérapie.

Un appareil de radiothérapie pour le traitement de tumeurs cancéreuses procède par irradiation des tumeurs au moyen de photons de haute énergie, émis par une source naturelle ou artificielle. Ces appareils sont en général très lourds, et utilisés dans des enceintes closes et blindées pour la protection du personnel, ce qui rend difficile la communication entre le patient et le médecin pendant les séances de traitement.

Par ailleurs, les critères d'efficacité des appareils de radiothérapie sont appréciés d'après deux effets biologiques bien définis :
- un effet létal, obtenu par concentration de faisceaux de photons assurant une dose déterminée dans un volume cible,
- un effet dit différentiel, obtenu par diffusion de faisceaux de photons dans un volume plus important que la cible, assurant une dose plus faible, pour permettre aux cellules saines de se régénérer.

Le document US 2,638,554 décrit un dispositif de guidage de photons pour appareil de radiothérapie, constitué par un corps en forme de tronc de cône, traversé par des canaux cylindriques convergeant tous au sommet du cône. Ce document ne fait pas mention de l'effet différentiel.

Le document US 2003/0209677 A1 décrit un autre dispositif de guidage de photons.

L'un des buts de l'invention est de proposer un dispositif de guidage de photons pour appareil de radiothérapie qui permette de conjuguer les deux effets, létal et différentiel.

Un autre but de l'invention est de proposer un dispositif de guidage de photons pour appareil de radiothérapie suffisamment simple et léger pour permettre l'utilisation de sources de rayonnement de faible énergie.

L'invention a pour objet un dispositif de guidage de photons pour appareil de radiothérapie caractérisé en ce qu'il est constitué d'un tronc de cône central constitué de plusieurs canaux métalliques, et d'un corps extérieur entourant le tronc de cône central et constitué de micro-canaux, lesdits canaux et micro-canaux ayant une forme tronconique et un sommet commun.

Avantageusement, lesdits canaux métalliques remplissent le volume du tronc de cône central en empilement compact.

Selon une caractéristique, le corps extérieur est réalisé par enroulement en spirale d'une feuille métallique sur laquelle sont disposés les micro-canaux.

Avantageusement, la feuille métallique est constituée par une bande à courbure circulaire, comprise entre deux arcs de cercle concentriques et deux bords latéraux définis par deux rayons issus du même centre.

Selon un mode de réalisation, chaque micro-canal est en forme de gouttière avec un fond appliqué sur la feuille métallique et deux ailes légèrement convergentes.

Avantageusement, l'enroulement en spirale de la feuille métallique est réalisé sur un support constitué par un premier tronc de cône mince ayant les dimensions extérieures du tronc de cône central.

L'enroulement en spirale de la feuille métallique est réalisé avec le côté ouvert des micro-canaux tourné vers le premier tronc de cône mince.

Avantageusement, pour son enroulement en spirale, la feuille métallique est bordée par deux ménisques massifs, en métal lourd.

A titre d'exemple, l'invention est décrite ci-après avec référence au dessin annexé dans lequel :
- la Figure 1 est une vue en perspective d'un exemple de réalisation d'un dispositif de guidage de photons selon l'invention,
- la Figure 2 est une vue de la feuille métallique découpée servant de support pour la réalisation du dispositif de guidage de photons de la figure 1,
- la Figure 3 est une vue de dessus du dispositif de la figure 1 montrant l'enroulement de la feuille de la figure 2,
- la Figure 4 est une vue de dessus agrandie d'un micro-canal avec vue en coupe.

En se reportant aux Figures, le dispositif 1 de guidage de photons est de forme sensiblement tronconique, et composé d'un tronc de cône central 2 et d'un corps extérieur 3 tronconique creux.

Le tronc de cône central 2 est constitué de plusieurs canaux métalliques 4, juxtaposés, tronconiques, ayant un sommet 5 commun. Ces canaux 4 remplissent le volume du tronc de cône central 2 en empilement compact.

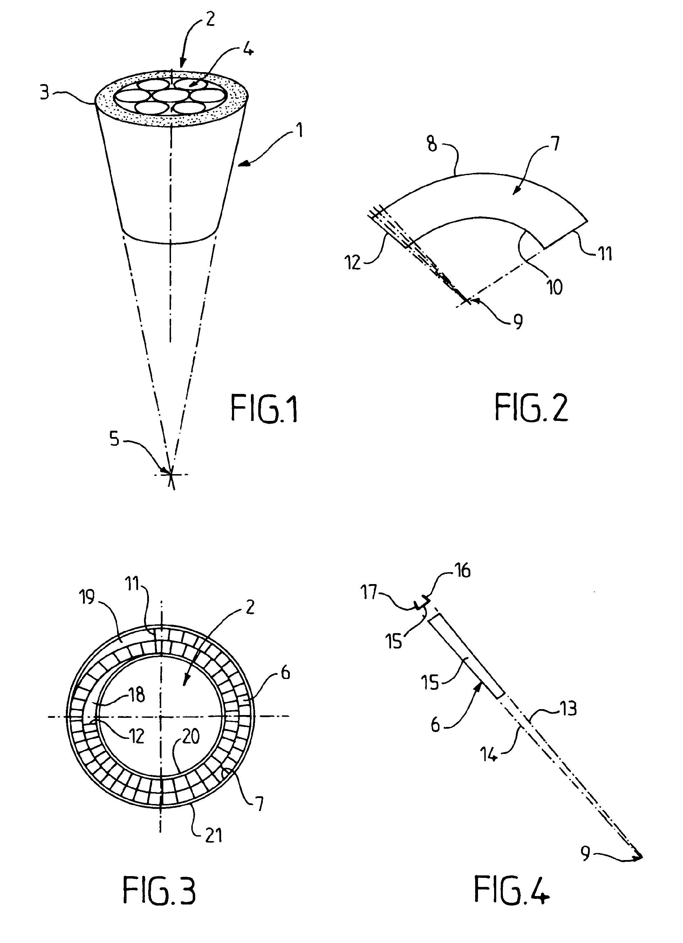

Le corps extérieur 3 est constitué d'un ensemble de micro-canaux 6 ayant le même sommet 5 commun que les canaux 4 du tronc de cône central 2. Ces micro-canaux 6 sont réalisés individuellement et fixés, par collage par exemple, sur une feuille métallique 7 découpée à plat (Fig. 2) dont les bords sont constitués d'une part par deux arcs de cercle concentriques et d'autre part par deux portions de rayon. Les deux arcs de cercle concentriques sont : l'un l'arc extérieur 8, de rayon R, tracé à partir du centre 9 ; l'autre l'arc intérieur 10 de rayon r inférieur à R, tracé à partir du même centre 9. Les deux autres bords, ou bords latéraux de la feuille métallique 7 ont pour longueur la différence (R-r) des deux rayons R et r. Ces deux bords 11 et 12 sont définis par deux rayons issus du même centre 9. Ils correspondent à la largeur de la feuille 7 qui a la forme d'une bande plane à courbure circulaire.

Chaque micro-canal 6 est disposé sur la feuille métallique plane 7, et il occupe sur la feuille la surface comprise entre deux rayons voisins 13, 14 passant par le centre 9. Chaque micro-canal 6 est en forme de gouttière, avec un fond 15 et deux ailes 16, 17. Le fond 15 est plat et appliqué sur la feuille 7. La longueur du fond 15 est égale à la largeur de la feuille 7, soit (R-r).

Les bords longitudinaux du fond 15 sont définis par deux rayons issus du centre 9. De ce fait, la largeur du fond 15 est décroissante entre le bord extérieur 8 et le bord intérieur 10 de la feuille 7. Les ailes 16 et 17 de chaque micro-canal 6 s'élèvent à partir des bords longitudinaux du fond. Leur hauteur est limitée par deux rayons issus du centre 9, elle est du même ordre de grandeur que la largeur du fond 15.

Les deux ailes 16, 17 d'un micro-canal 6 ne sont pas parallèles entre elles mais légèrement convergentes en vue de l'enroulement ultérieur décrit ci-après.

Les micro-canaux 6 sont juxtaposés sur la feuille 7, leurs fonds étant adjacents et couvrant la surface de la feuille 7. Les ailes voisines de deux micro-canaux 6 adjacents sont légèrement divergentes sur la feuille 7 encore à plat.

Pour la réalisation du corps extérieur 3, on utilise comme supports deux troncs de cône minces 20, 21, en acier inoxydable, ayant le même sommet 5. Le premier tronc de cône mince 20 a sensiblement les dimensions extérieures du tronc de cône central 2. Le deuxième tronc de cône mince 21 a les dimensions extérieures du corps extérieur 3.

Sur le premier tronc de cône mince 20 sont enroulés successivement : un premier ménisque 18 massif en métal lourd tel que le plomb ou l'or, la feuille 7 avec les micro-canaux 6 dont le côté ouvert est tourné vers le premier tronc de cône mince19, et un second ménisque 19 de même nature que le premier. La feuille 7 munie des micro-canaux 6 et les deux ménisques 18, 19 bordant la feuille 7 constituent des composants déformables et jointifs dans le corps extérieur 3.

Au cours de cet enroulement en spirale, les ailes voisines de deux micro-canaux 6 adjacents se rapprochent et arrivent en contact réciproque. Une fois l'enroulement terminé, l'ensemble est placé dans le deuxième tronc de cône mince 21.

Le tronc de cône central 2 est mis en place dans le corps extérieur 3 pour achever la constitution du dispositif de guidage de photons.

Dans le dispositif de guidage de photons ainsi constitué, les canaux métalliques 4 du tronc de cône central 2 et les micro-canaux 6 du corps extérieur 3 ont une forme tronconique et un sommet commun 5.

Le dispositif de guidage de photons est utilisé dans un appareil de radiothérapie pour irradier une cible placée au sommet 5 (Fig. 1).

Dans ce cas, la source radioactive est placée à l'extérieur du dispositif de guidage 1 de photons, sur la surface de la grande base du tronc de cône.

La grande surface disponible permet d'utiliser une source avec un isotope radioactif d'activité spécifique moyenne, mais d'intensité globale importante.

Les canaux 4 du tronc de cône central 2 guident les photons qui assurent l'effet différentiel. Ces canaux 4 utilisent des moyens techniques usuels de filtrage et de collimation large. Les micro-canaux 6 du corps extérieur 3 guident avec une grande précision les photons qui assurent l'effet létal. La disposition du tronc de cône central 2 et du corps extérieur 3, avec des structures différentes pour les canaux 4 et les micro-canaux 6, permet d'assurer simultanément l'irradiation à effet différentiel et l'irradiation à effet létal.

La disposition des micro-canaux 6 permet de laisser passer les photons qui sont dans l'axe des micro-canaux en arrêtant les autres, ce qui évite le risque d'un effet cancérigène collatéral.

Le dispositif de guidage de photons a été décrit avec une forme générale en tronc de cône. Toutefois, lorsque le corps extérieur 3 est d'une certaine épaisseur, la forme générale est plutôt celle d'une portion de sphère, la grande surface, extérieure, étant sphérique convexe et la petite surface, intérieure, étant sphérique concave, les canaux et micro-canaux convergeant au centre, unique, des deux surfaces sphériques.

A titre d'exemple, pour des énergies de photons de 100 à 500 keV, la feuille métallique a une épaisseur de 0,3 mm, le rayon R est de 14 cm, le rayon r de 9 cm, la largeur de la feuille, ou la hauteur du dispositif de guidage, est de 5 cm, et les micro-canaux ont une dimension intérieure de l'ordre de 1,5 mm. De préférence, le dispositif de guidage de photons est réalisé en or.

Dans le cas d'une application du dispositif de guidage de photons à un appareil de radiothérapie utilisé pour le cancer du sein, où la profondeur de pénétration des photons est très faible, un radio-isotope à émission de faible énergie est adapté à la majorité des cas : il s'agit du thulium 170, qui a une émission proche de celles utilisées en mammographie. Dans ce cas, la taille du dispositif de guidage de photons est très faible et permet une balistique d'irradiation par circulation autour du sein, ce qui évite l'apparition du cancer du sein dit second, ou induit.

## Revendications

1. Dispositif de guidage de photons pour appareil de radiothérapie constitué d'un tronc de cône central (2) constitué de plusieurs canaux métalliques (4), remplissant le volume du tronc de cône central (2) en empilement compact, et d'un corps extérieur (3) entourant le tronc de cône central et constitué de micro-canaux (6), lesdits canaux (4) et micro-canaux (6) ayant une forme tronconique et un sommet commun (5), **caractérisé en ce que** le corps extérieur (3) est réalisé par enroulement en spirale d'une feuille métallique (7) sur laquelle sont disposés les micro-canaux (6).

2. Dispositif de guidage de photons selon la revendication 1, **caractérisé en ce que** la feuille métallique est constituée par une bande à courbure circulaire, comprise entre deux arcs de cercle concentriques (8, 10) et deux bords latéraux (11, 12) définis par deux rayons issus du même centre.

3. Dispositif de guidage de photons selon la revendication 1, **caractérisé en ce que** chaque micro-canal (6) est en forme de gouttière avec un fond (15) appliqué sur la feuille métallique (7) et deux ailes (16, 17) légèrement convergentes.

4. Dispositif de guidage de photons selon la revendication 1, **caractérisé en ce que** les micro-canaux (6) sont juxtaposés sur la feuille métallique (7).

5. Dispositif de guidage de photons selon la revendication 1, **caractérisé en ce que** l'enroulement en spirale de la feuille métallique (7) est réalisé sur un support constitué par un premier tronc de cône mince (20) ayant les dimensions extérieures du tronc de cône central (2).

6. Dispositif de guidage de photons selon la revendication 5, **caractérisé en ce que** l'enroulement en spirale de la feuille métallique (7) est réalisé avec le côté ouvert des micro-canaux (6) tourné vers ledit premier tronc de cône mince (20).

7. Dispositif de guidage de photons selon la revendication 6, **caractérisé en ce que** pour son enroulement en spirale, la feuille métallique (7) est bordée par deux ménisques (18, 19) massifs, en métal lourd.

## Patentansprüche

1. Photonenlenkvorrichtung für ein Strahlentherapiegerät, die von einem zentralen Kegelstumpf (2), den mehrere metallische Kanäle (4) bilden, die das Volumen des zentralen Kegelstumpfs (2) als kompakte Stapelung ausfüllen, und von einem äußeren Körper (3), der den zentralen Kegelstumpf umgibt und von Mikrokanälen (6) gebildet ist, gebildet ist, wobei die Kanäle (4) und Mikrokanäle (6) eine Kegelstumpfform haben und eine gemeinsame Spitze (5), **dadurch gekennzeichnet, dass** der äußere Körper (3) durch spiralförmiges Wickeln einer Metallfolie (7) gebildet ist, auf der die Mikrokanäle (6) angeordnet sind.

2. Photonenlenkvorrichtung nach Anspruch 1, **dadurch gekennzeichnet, dass** die Metallfolie von einem Streifen mit kreisrunder Krümmung gebildet ist, der zwischen zwei konzentrischen Kreisbögen (8, 10) und zwei Seitenrändern (11, 12), die von zwei Radien definiert sind, die im selben Zentrum ihren Ursprung haben, inbegriffen ist.

3. Photonenlenkvorrichtung nach Anspruch 1, **dadurch gekennzeichnet, dass** jeder Mikrokanal (6) mit einem auf der Metallfolie (7) anliegenden Boden (15) und zwei leicht konvergierenden Flügeln (16, 17) wie eine Rinne geformt ist.

4. Photonenlenkvorrichtung nach Anspruch 1, **dadurch gekennzeichnet, dass** die Mikrokanäle (6) auf der Metallfolie (7) aneinandergereiht sind.

5. Photonenlenkvorrichtung nach Anspruch 1, **dadurch gekennzeichnet, dass** die Spiralwicklung der Metallfolie (7) auf einem Träger durchgeführt ist, der von einem ersten schlanken Kegelstumpf (20) gebildet ist, der die Außenmaße des zentralen Kegelstumpfs (2) hat.

6. Photonenlenkvorrichtung nach Anspruch 5, **dadurch gekennzeichnet, dass** die Spiralwicklung der Metallfolie (7) mit der geöffneten Seite der Mikrokanäle (6) durchgeführt wird, die in Richtung des ersten schlanken Kegelstumpfs (20) zeigt.

7. Photonenlenkvorrichtung nach Anspruch 6, **dadurch gekennzeichnet, dass** die Metallfolie (7) für ihre Spiralwicklung von zwei massiven Sicheln (18, 19) aus Schwermetall gesäumt ist.

## Claims

1. A photon-guiding device for a radiotherapy apparatus made up of a truncated central cone (2) formed by several metal channels (4), filling the volume of the truncated central cone (2) in a compact stack, and an outer body (3) surrounding the truncated central cone and made up of micro-channels (6), said channels (4) and micro-channels (6) having a frustoconical shape and a shared apex (5), **characterized in that** the outer body (3) is produced by a spiral winding of a metal foil (7) over which the micro-channels (6) are arranged.

2. The photon-guiding device according to claim 1, **characterized in that** the metal foil is made up of a strip with a circular curvature, comprised between two concentric arcs of circle (8, 10) and two lateral edges (11, 12) defined by two radii coming from the same center.

3. The photon-guiding device according to claim 1, **characterized in that** each micro-channel (6) is in the form of a trough with a bottom (15) applied on the metal foil (7) and two slightly converging wings (16, 17).

4. The photon-guiding device according to claim 1, **characterized in that** the micro-channels (6) are juxtaposed on the metal foil (7).

5. The photon-guiding device according to claim 1, **characterized in that** the spiral winding of the metal foil (7) is done on a support made up of a first thin truncated cone (20) having the outer dimensions of the truncated central cone (2).

6. The photon-guiding device according to claim 5, **characterized in that** the spiral winding of the metal foil (7) is done with the open side of the micro-channels (6) turned toward said first thin truncated cone (20).

7. The photon-guiding device according to claim 6, **characterized in** for its spiral winding, the metal foil (7) is bordered by two massive meniscuses (18, 19), made from heavy metal.
